(19) Europäisches Patentamt

European Patent Office

Office européen des brevets

(11) Veröffentlichungsnummer: **0 343 548 B1**

# (12) EUROPÄISCHE PATENTSCHRIFT

(45) Veröffentlichungstag der Patentschrift: **15.12.93**

(21) Anmeldenummer: **89109127.4**

(22) Anmeldetag: **20.05.89**

(51) Int. Cl.⁵: **A61K 39/39**, A61K 39/00, A61K 47/00

Die Akte enthält technische Angaben, die nach dem Eingang der Anmeldung eingereicht wurden und die nicht in dieser Patentschrift enthalten sind.

(54) **Antigenlösung ethaltend ein Polyalphaolefin (PAO), Verfahren ihrer Herstellung und die Verwendung von PAO als Adjuvans.**

(30) Priorität: **24.05.88 DE 3817531**

(43) Veröffentlichungstag der Anmeldung:
**29.11.89 Patentblatt 89/48**

(45) Bekanntmachung des Hinweises auf die Patenterteilung:
**15.12.93 Patentblatt 93/50**

(84) Benannte Vertragsstaaten:
**AT BE CH DE ES FR GB GR IT LI LU NL SE**

(56) Entgegenhaltungen:
**EP-A- 0 187 286**

**CHEMICAL ABSTRACTS, Band 99, Nr. 23, 05 Dezember 1983, Columbus, OH (US); P.D. GUINEY, p. 237, no. 189126d&NUM;**

(73) Patentinhaber: **BEHRINGWERKE Aktiengesellschaft**
**Postfach 1140**
**D-35001 Marburg(DE)**

(72) Erfinder: **Bernhardt, Dieter, Dr.**
**Goldbergstrasse 18A**
**D-3553 Cölbe(DE)**

EP 0 343 548 B1

**Beschreibung**

Die Erfindung betrifft Antigenlösungen enthaltend ein Polyalphaolefin (PAO), besonders Vaccinen, Verfahren ihrer Herstellung und die Verwendung eines PAO als Adjuvans in Vaccinen.

Viele Antigene sind so wenig immunogen, daß sie bei einmaliger Injektion in ein Tier oder einen Menschen keine meßbare oder nur eine geringe Immunantwort auslösen. Um die Immunantwort des Organismus auf einen Antigenreiz zu verstärken, werden deshalb dem Antigen Adjuvantien zugesetzt. Die meisten inaktivierten Virus- und Bakterienvaccinen enthalten aus diesem Grund Adjuvantien.

In den erwähnten Vaccinen benutzt man überwiegend A1(OH)$_3$ oder A1PO$_4$, auch in Kombination, Pflanzenöle oder aus Erdölfraktionen gewonnene Mineralöle, sogenannte medizinisch pharmazeutische Weißöle, nicht exakt definierter Zusammensetzung.

All diesen aufgeführten Adjuvantien ist aber gemeinsam, daß sie neben Lokalreaktionen auch systemische Nebenwirkungen entfalten.

Überraschenderweise wurde nun gefunden, daß stark verzweigte isoparaffinische Polyalphaolefine (Typ: [R]Synfluid PAO) eine gute Adjuvanswirkung entfalten, ohne die beträchtlichen Nebenwirkungen der letzterwähnten Adjuvantien zu bewirken.

Gegenstand der Erfindung ist daher eine wässrige Antigenlösung, besonders eine Vaccine, enthaltend eine adjuvierend wirksame Menge eines Polyalphaolefins und einen Emulgator.

Solche Polyalphaolefine sind besonders solche, die als "highly branched isoparaffinic polyalphaolefins" bezeichnet und unter der Bezeichnung [R]Synfluid (Chevron Oil Co., USA) verfügbar sind.

Solche Antigenlösungen enthalten das PAO in emulgierter Form, wozu ein Emulgator, vorzugsweise ein Polyoxyäthylen-(20)-Sorbitan Monooleat ([R]Tween 80) oder/und Polyoxyäthylen-(5)-Sorbitan Monooleat ([R]Tween 81) zugesetzt wird.

Das Volumenverhältnis von wässriger Antigenlösung zur Emulsion von PAO und Emulgator beträgt 0,05-0,3 vorzugsweise 0,1-0,2.

Das Volumenverhältnis PAO/Emulgator in der Emulsion ist 10:0.5 bis 10:3, vorzugsweise 10:1.

Gegenstand der Erfindung ist auch Verfahren zur Herstellung einer Emulsions-Vaccine, dadurch gekennzeichnet, daß 6,5-98,99 Volumteile einer wässrigen Antigenlösung, 1-85 Volumteile eines Polyalphaolefins und 0,01-8,5 Volumteile eines Emulgators emulgiert werden.

Verfahren zum Adjuvieren von wässrigen Antigenlösungen durch Emulgieren mit Mineral- oder Pflanzenölen sind bekannt.

Für die Herstellung einer Emulsionsvaccine sind prinzipiell 3 Verfahren bekannt:
1. Herstellung einer Wasser in Öl-Emulsion (W/O)
2. Herstellung einer Öl in Wasser-Emulsion (O/W)
3. Herstellung einer Doppelemulsion (DE)
Die DE kann in zweierlei Weise hergestellt werden:
   a) Zuerst als W/O- und danach als O/W-Emulsion
   b) Zuerst als O/W- und danach als W/O-Emulsion
Weil PAO in wässrigen Lösungen nicht löslich ist, ist es notwendig, PAO zu emulgieren, um eine gleichmäßige Verteilung von Antigen und PAO herzustellen, wozu ein Emulgator zugesetzt werden muß. Hierbei erwiesen sich [R]Tween 80 und [R]Tween 81 als besonders geeignete Emulgatoren. Mit dieser Emulgatorkombination gelingt es, stabile W/O, O/W oder DE vom Typ W/O-O/W-Emulsionen herzustellen.

Die Prozentangaben im folgenden bedeuten Volumprozente. Um eine erfindungsgemäße Antigenlösung als W/O-Emulsion herzustellen, kann folgendermaßen gearbeitet werden:
   a) Vorlage von 1 bis 85% PAO
   b) unter Emulgierbedingungen, vorzugsweise [R]Ultraturax (UT)-Behandlung wird ein Emulgator, vorzugsweise 0,0075 - 6,38% [R]Tween 81 und
   c) gegebenenfalls 0,0025 - 2,12% [R]Tween 80 zugemischt
   d) unter Emulgierbedingungen werden 98,99 - 6,5% wässrige Antigenlösung zugemischt, und anschließend wird die Emulsion 3 - 5 Min. weiter kräftig durchgemischt.

Verwendet man nur geringe Konzentrationen von PAO, ist es möglich, die Emulgatorkonzentration zu verdoppeln oder zu verdreifachen.

Als Antigene, die mit PAO im Sinne der Erfindung vorteilhaft adjuviert werden können, kommen Vaccine-Antigene wie Virus-, Bakterien-, Zellwand- und Proteinantigene in Frage.

Um eine erfindunsgemäße Antigenlösung als O/W-Emulsion herzustellen, kann folgendermaßen gearbeitet werden:
   a) Vorlage von 1 - 85% PAO
   b) unter Emulgierbedingungen werden 0,0075 - 6,38% Emulgator, vorzugsweise [R]Tween 81 zugemischt

2

c) Vorlage von 98,99 - 6,5% Antigenlösung

d) unter Emulgierbedingungen werden 0,0025 - 2,12% Emulgator, vorzugsweise [R]Tween 80 in die Antigenlösung eingemischt, danach wird

e) das Gemisch aus PAO und [R]Tween 81 unter Emulgierbedingungen in das Antigen-[R]Tween 80-Gemisch zur fertigen O/W-Emulsion eingemischt.

Um eine erfindungsgemäße Antigenlösung als Doppelemulsion vom Typ W/O - O/W herzustellen, kann folgendermaßen gearbeitet werden:

I.

a) Vorlage von 1 - 85 % PAO

b) unter Emulgierbedingungen werden 0,0075 % - 6,38 % Emulgator, vorzugsweise [R]Tween 81 zugemischt

c) unter Emulgierbedingungen werden 0,5 % - 50 % Antigenlösung zugemischt.

II.

a) Vorlage von 98,4 % - 6,0 % Antigenlösung

b) unter Emulgierbedingungen werden 0,0025 % - 2,12 % Emulgator, vorzugsweise [R]Tween 80 zugemischt, danach wird

III. die W/O-Emulsion von I unter Emulgierbedingungen in das Antigen/[R]Tween 80 Gemisch eingemischt und anschließend wird die Emulsion 3 - 5 Min. weiter kräftig durchgemischt.

Die Antigenlösungen, Emulsionen und Vaccinen werden bei 4 °C bis 30 °C gemischt. Der pH-Wert der Vaccinen liegt bei pH 7,2-8,5.

Beispiele

Beispiel 1

Aus inaktiviertem Aujeszkyvirusantigen (AV-Antigen) wurden 2 verschiedene Vaccinen hergestellt.
Vaccine A war eine Wasser in Öl-Emulsion (W/O) folgender Zusammensetzung:

| | |
|---|---|
| PAO, 6 cps, MG, 500 (Chevron Oil Co., Oronite Additives Division, 575 Market St., San Francisco, CA. 94105, USA) | 45,0 ml |
| Tween 81 | 6,75 ml |
| Tween 80 | 2,25 ml |
| AV-Antigen | 46,0 ml |
| | 100,0 ml |

Vaccine B (Referenzvaccine) war eine W/O Emulsion folgender Zusammensetzung:

| | |
|---|---|
| [R]Marcol 52 (ein Mineralöl) | 45,0 ml |
| [R]Montanide 888 | 5,0 ml |
| AV-Antigen | 50,0 ml |
| | 100,0 ml |

Beide W/O Vaccinen waren nach Emulgierung stabil, d.h. es kam zu keiner Phasentrennung zwischen der wässrigen und öligen Phase.

Mit beiden Vaccinen wurden jeweils 2 Schweine, 30 - 35 kg schwer, mit 2,0 ml s.c. vacciniert. Die Antikörper gegen Aujeszkyvirus wurden vor Vaccination, 3 Wochen p.v. (nach Vaccination) und 7 Wochen p.v. im Serum der Tiere bestimmt. 7 Wochen p.v. wurden die Tiere und 2 Kontrollen mit infektiösem Virus belastet.

Die Ergebnisse des Schutzversuches sind nachfolgend tabellarisch aufgeführt.

Tabelle 1

| Vaccine | Lokale Verträglichkeit (Punkte) | | reziproker AujeszkyvirusAntikörper/0.1 ml Serum | | | Gewichtsentwicklung nach Belastung innerhalb von |
|---|---|---|---|---|---|---|
| | | | vor Vacc. | 3Wo.p.v. | 7Wo.p.v. | 11 Tagen |
| B | 286 N | kleiner als 1 | | 39 | 78 | + 11 % |
| | 266 N | " " 1 | | 22 | 68 | + 5 % |
| A | 76 | kleiner als 1 | | 45 | 118 | + 9 % |
| | 76 | " " 1 | | 39 | 78 | + 11 % |
| Kontrollen | 0 | | | | | − 18 % |
| | 0 | | | | | verendet 5 Tage p.i. |

N = Nekrose

+ = Gewichtszunahme

− = Gewichtsabnahme

Aus diesem Versuch geht klar hervor, daß eine W/O Emulsion mit PAO als Adjuvans (Vaccine A) wesentlich verträglicher ist (geringe Punktzahl) als eine Vaccine, die [R]Marcol 52 als Adjuvans enthält (hohe Punktzahl). Bei beiden Tieren der Vaccine B traten am Tag 13 p.v. bzw. am Tag 17 Nekrosen an der Injektionsstelle auf.
Die Entwicklung neutralisierender Antikörper ist bei beiden Vaccinen in etwa gleich.
Nach der Belastungsinfektion wiesen sowohl die mit Vaccine A als auch mit Vaccine B geimpften Tiere eine gute Gewichtsentwicklung auf.
Eine Kontrolle verendete 5 Tage p.i. Bei der anderen Kontrolle waren deutliche Krankheitssymptome der Aujeszkyschen Krankheit und starke Gewichtsabnahme (-18%) ausgeprägt.

Beispiel 2

Aus inaktiviertem AV-Antigen wurden 3 Vaccinen mit unterschiedlichem PAO-Gehalt als Doppelemulsionen (DE) hergestellt.

| | Vaccine A | Vaccine B | Vaccine C |
|---|---|---|---|
| PAO | 40,0 ml | 20,0 ml | 10,0 ml |
| [R]Tween 81 | 3,0 ml | 1,5 ml | 0,75 ml |
| [R]Tween 80 | 1,0 ml | 0,5 ml | 0,25 ml |
| AV-Antigen | 56,0 ml | 78,0 ml | 89,0 ml |

Mit diesen 3 Vaccinen wurden jeweils 2 Schweine, 30 - 35 kg schwer, mit 2,0 ml s.c. vacciniert. Die neutralisierenden Antikörper gegen das Aujeszkyvirus wurden vor Vaccination, 3 Wochen p.v. und 7 Wochen p.v. im Serum der Tiere bestimmt. Die Ergebnisse dieses Immunisierungsversuches sind nachfolgend tabellarisch aufgeführt.

## Tabelle 2

| Vaccine | Lokale Verträglichkeit (Punkte) | reziproker AV-Antikörpertiter/0.1 ml | | | Schlachtbefund 7 Wochen p.v. |
|---|---|---|---|---|---|
| | | vor Vacc. | 3Wo.p.v. | 7Wo.p.v. | |
| A | 4 | kleiner als 1 | 20 | 68 | * |
| | 11 | " " 1 | 18 | 30 | |
| B | 10 | kleiner als 1 | 18 | 30 | o.B. |
| | 10 | " " 1 | 8 | 22 | o.B |
| C | 10 | kleiner als 1 | 12 | 34 | o.B. |
| | 0 | " " | 4 | 17 | o.B. |

\* = 3 stecknadelkopfgroße, grießliche Herde an der Injektionsstelle 1 mm breite, 3 cm langer Strang von lymphknotenartiger Konsistenz an der Impfstelle

Auch dieser Versuch zeigt, daß eine DE mit PAO als Adjuvans, sehr gut verträglich und wirksam ist, wobei die Antikörpertiter von der 3. Woche p.v. zur 7. Woche p.v. noch ansteigen.

Beispiel 3

Aus inaktiviertem Parainfluenza, Typ 3 (PI$_3$)- und Infektiöse Bovine Rhinotracheitis (IBR)-Antigen wurden Vaccinen hergestellt
Vaccine A mit 20 % PAO als DE
Vaccine B mit 10 % PAO als DE
Vaccine C mit 5 % PAO als DE
Vaccine D mit 10 % Al(OH)$_3$
Mit jeder Vaccine wurden 3 Hunde mit je 1 ml Vaccine s.c. vacciniert. 3 Wochen p.v. wurden die Tiere mit gleicher Dosis revacciniert. Im Serum der Tiere wurden die neutralisierenden Antikörper gegen PI$_3$ und IBR Virus vor Vaccination, 3 Wochen p.v. (vor 2. Vaccination) und 1 Woche nach Revaccination bestimmt.
Die Resultate dieses Versuches sind nachfolgend tabellarisch aufgeführt (Tabelle 3).
Dieser Versuch zeigt klar, die gute Verträglichkeit von PAO-haltigen Vaccinen am Hund nach 2maliger Applikation. Die Al(OH)$_3$ enthaltende Vaccine ist lokal wesentlich unverträglicher. Die Lokalreaktion der 1. und 2. Impfung war bei Versuchsende noch nicht abgeklungen ( + ). Hinsichtlich der Wirksamkeit bestehen zwischen den 4 Vaccinen keine signifikanten Unterschiede.

Beispiel 4

10 Mäuse wurden mit 0,1 ml nativem Kälberserum s.c. vacciniert, weitere 10 Mäuse wurden mit 0,1 ml nativem Kälberserum, das 50 % PAO in DE enthielt, s.c. vacciniert. 6 Wochen p.v. wurden die Tiere entblutet, das Serum gewonnen, gepoolt und in der Agargelpräzipitation (AGP) auf Antikörper untersucht. Im Mäuseserum, die nur natives Kälberserum erhalten hatten, wurde ein AGP-Titer von 1:2 nachgewiesen. Im Mäuseserum, die natives Kälberserum mit PAO als Adjuvans erhielten, war ein AGP-Titer von 1:64 feststellbar.

Beispiel 5

Aus inaktivierten Pasteurella spezifischem Antigen wurden 2 Vaccinen hergestellt.
Vaccine A enthielt 60 % PAO in DE.

**Tabelle 3**

| Vaccine | Lokale Verträglich-keit (Punkte) | | reziproker Antikörpertiter/0,1 ml Serum | | | | | |
|---|---|---|---|---|---|---|---|---|
| | 1.Vacc. | 2.Vacc. | vor Vacc. | 3Wo.p.v. | 1Wo.p.rev. | vor Vacc. | 3Wo p.v. | 1Wo.p.rev. |
| A | 0 | 0 | kleiner als 1 | 8 | 204 | kleiner als 14 | 28 | * 3548 |
| | 0 | 0 | " " 1 | 12 | 260 | " " 14 | 28 | * 3548 |
| | 0 | 0 | " " 1 | 8 | 312 | " " 14 | 42 | * 3548 |
| B | 0 | 0 | " " 1 | 6 | 208 | " " 14 | 42 | * 3548 |
| | 0 | 0 | " " 1 | 4 | 201 | " " 14 | 42 | * 3548 |
| | 0 | 0 | " " 1 | 4 | 212 | " " 14 | 28 | * 2344 |
| C | 0 | 0 | " " 1 | 5 | 182 | " " 14 | 21 | * 3548 |
| | 0 | 0 | " " 1 | 3 | 208 | " " 14 | 21 | * 2344 |
| | 0 | 0 | " " 1 | 2 | 201 | " " 14 | 28 | * 2692 |
| D | 5 | 10 | " " 1 | 11 | 176 | " " 14 | 21 | * 3548 |
| | 11 | 15 | " " 1 | 7 | 184 | " " 14 | 21 | * 2692 |
| | 12,5 | 13 | " " 1 | 6 | 220 | " " 14 | 42 | * 2344 |

* = größer oder gleich

7

Vaccine B enthielt nur Pasteurella Antigen.

Mit beiden Vaccinen wurden jeweils 10 Mäuse mit je 0,2 ml s.c. vacciniert. Die Tiere wurden 6 Wochen p.v. entblutet. Im Serum war bei Vaccine A ein HAH (Hämagglutinationshemmung)-Titer von 1:2048 nachweisbar, währen in der Vaccine B nur ein HAH-Titer von 1:32 aufzufinden war.

Dieser Versuch zeigt die sehr gute adjuvierende Wirkung von PAO. Obwohl die Vaccine A 66 % weniger Antigen enthält, ist der HAH-Titer ganz wesentlich höher, als bei der nichtadjuvierten Vaccine B.

**Patentansprüche**
**Patentansprüche für folgende Vertragsstaaten : AT, BE, CH, DE, FR, GB, IT, LI, LU, NL, SE**

1. Wässerige Antigenlösung, enthaltend ein Polyalphaolefin (PAO) und einen Emulgator.

2. Antigenlösung nach Anspruch 1, dadurch gekennzeichnet, daß der Emulgator Polyoxyäthylen-(20)-Sorbitan Monooleat und/oder Polyoxyäthylen-(5)-Sorbitan Monooleat ist.

3. Antigenlösung nach Anspruch 1, dadurch gekennzeichnet, daß das Volumenverhältnis von wässriger Antigenlösung zur Emulsion von PAO und Emulgator 0,05-0,3 vorzugsweise 0,1-0,2 beträgt.

4. Antigenlösung nach Anspruch 1, dadurch gekennzeichnet, daß das Volumenverhältnis PAO/Emulgator in der Emulsion 10:0.5 bis 10:3, vorzugsweise 10:1 beträgt.

5. Verfahren zur Herstellung einer Emulsions-Vaccine, dadurch gekennzeichnet, daß eine wässrige Antigenlösung, ein Polyalphaolefin und ein Emulgator emulgiert werden.

6. Verfahren zur Herstellung einer Emulsions-Vaccine, dadurch gekennzeichnet, daß 6,5-98,99 Volumteile einer wässrigen Antigenlösung, 1-85 Volumteile eines Polyalphaolefins und 0,01-8,5 Volumteile eines Emulgators emulgiert werden.

7. Verwendung eines Polyalphaolefins in einem Verfahren zur Herstellung einer Vaccine.

**Patentansprüche für folgende Vertragsstaaten : ES, GR**

1. Verfahren zur Herstellung einer Emulsions-Vaccine, dadurch gekennzeichnet, daß eine wässrige Antigenlösung, ein Polyalphaolefin und ein Emulgator emulgiert werden.

2. Verfahren zur Herstellung einer Emulsions-Vaccine, dadurch gekennzeichnet, daß 6,5-98,99 Volumteile einer wässrigen Antigenlösung, 1-8,5 Volumteile eines Polyalphaolefins und 0,01-8,5 Volumteile eines Emulgators emulgiert werden.

**Claims**
**Claims for the following Contracting States : AT, BE, CH, DE, FR, GB, IT, LI, LU, NL, SE**

1. An aqueous antigen solution containing a polyalphaolefin (PAO) and an emulsifier.

2. An antigen solution as claimed in claim 1, wherein the emulsifier is polyoxyethylene (20) sorbitan monooleate and/or polyoxyethylene (5) sorbitan monooleate.

3. An antigen solution as claimed in claim 1, wherein the ratio by volume of aqueous antigen solution to the emulsion of PAO and emulsifier is 0.05-0.3, preferably 0.1-0.2.

4. An antigen solution as claimed in claim 1, wherein the PAO/emulsifier ratio by volume in the emulsion is 10:0.5 to 10:3, preferably 10:1.

5. A process for preparing an emulsion vaccine, which comprises emulsifying an aqueous antigen solution, a polyalphaolefin and an emulsifier.

6. A process for preparing an emulsion vaccine, which comprises emulsifying 6.5-98.99 parts by volume of an aqueous antigen solution, 1-85 parts by volume of a polyalphaolefin and 0.01-8.5 parts by volume of an emulsifier.

7. The use of a polyalphaolefin in a process for preparing a vaccine.

**Claims for the following Contracting States : ES, GR**

1. A process for preparing an emulsion vaccine, which comprises emulsifying an aqueous antigen solution, a polyalphaolefin and an emulsifier.

2. A process for preparing an emulsion vaccine, which comprises emulsifying 6.5-98.99 parts by volume of an aqueous antigen solution, 1-85 parts by volume of a polyalphaolefin and 0.01-8.5 parts by volume of an emulsifier.

**Revendications**

**Revendications pour les Etats contractants suivants : AT, BE, CH, DE, FR, GB, IT, LI, LU, NL, SE**

1. Solution aqueuse d'antigène contenant une poly-alphaoléfine (PAO) et un émulsifiant.

2. Solution d'antigène selon la revendication 1,
caractérisée en ce que l'émulsifiant est un mono-oléate de polyoxyéthylène-(20)-sorbitan et/ou un mono-oléate de polyoxyéthylène-(5)-sorbitan.

3. Solution d'antigène selon la revendication 1,
caractérisée en ce que le rapport de solution aqueuse d'antigène à l'émulsion de PAO et d'émulsifiant vaut, en volume, de 0,05 à 0,3, de préférence, de 0,1 à 0,2.

4. Solution d'antigène selon la revendication 1,
caractérisée en ce que, dans l'émulsion, le rapport PAO/émulsifiant vaut, en volume, de 10:0,5 à 10:3, de préférence, 10:1.

5. Procédé pour préparer un vaccin en émulsion,
caractérisé en ce qu'on émulsionne une solution aqueuse d'antigène, une poly-alpha-oléfine et un émulsifiant.

6. Procédé pour préparer un vaccin en émulsion,
caractérisé en ce qu'on émulsionne de 6,5 à 98,99 parties en volume d'une solution aqueuse d'antigène, de 1 à 85 parties en volume d'une poly-alpha-oléfine et de 0,01 à 8,5 parties en volume d'un émulsifiant.

7. Utilisation d'une poly-alpha-oléfine dans un procédé pour préparer un vaccin.

**Revendications pour les Etats contractants suivants : ES, GR**

1. Procédé pour préparer un vaccin en émulsion,
caractérisé en ce qu'on émulsionne une solution aqueuse d'antigène, une poly-alpha-oléfine et un émulsifiant.

2. Procédé pour préparer un vaccin en émulsion,
caractérisé en ce qu'on émulsionne de 6,5 à 98,99 parties en volume d'une solution aqueuse d'antigène, de 1 à 85 parties en volume d'une poly-alpha-oléfine et de 0,01 à 8,5 parties en volume d'un émulsifiant.